(19) Europäisches Patentamt
European Patent Office
Office européen des brevets

(11) Publication number: 0 347 733 A1

(12) **EUROPEAN PATENT APPLICATION**

(21) Application number: 89110780.7

(22) Date of filing: 14.06.89

(51) Int. Cl.4: C07D 491/107 , A61K 31/415

(30) Priority: 20.06.88 JP 151715/88

(43) Date of publication of application: 27.12.89 Bulletin 89/52

(84) Designated Contracting States: AT BE CH DE FR GB IT LI NL SE

(71) Applicant: Eisai Co., Ltd.
6-10, Koishikawa 4-chome Bunkyo-ku
Tokyo(JP)

(72) Inventor: Minami, Norio
709-59, Shimohirooka
Tsukuba-shi Ibaraki(JP)
Inventor: Matsukura, Masayuki
25-5-602, Umezono 2-chome
Tsukuba-shi Ibaraki(JP)

(74) Representative: Hansen, Bernd, Dr.rer.nat. et al
Hoffmann, Eitle & Partner Patentanwälte
Arabellastrasse 4 Postfach 81 04 20
D-8000 München 81(DE)

(54) **Optically active hydantoin derivatives.**

(57) An optically active hydantoin compound, -6-halogen-2,2-dimethyl-1′-(3-(4-hydroxypiperidino)-propyl)spiro-(chroman-4,4′-imidazolidine)-2′,5′-dione, having the formula:

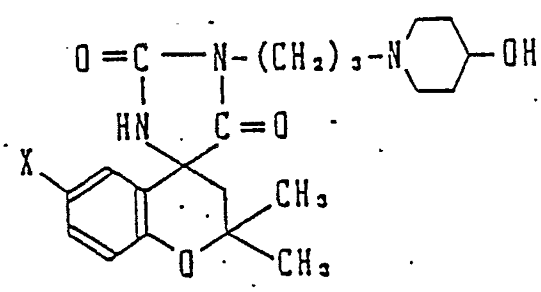

in which X is a halogen, and a pharmacologically acceptable acid addition salt thereof are novel and useful for the pharmacological use, such as an antiarrhythmia agent. X is preferably chlorine.

EP 0 347 733 A1

# OPTICALLY ACTIVE HYDANTOIN DERIVATIVES

The present invention relates to optically active hydantoin derivatives having an excellent medicinal effect.

[Prior Art ]

Although various antiarrythmic agents such as procaineamide, quinidine, disopyramide, ajmalin, lidocaine, phenytoin, mexiletine, aprindine and tocainide are known, their safety is yet unsatisfactory.

After intensive investigations made for the purpose of developing an antiarrythmic agent of a new type under these circumstances, the inventor previously found that hydantoin derivatives of following general formula (A) and acid addition salts thereof had an excellent antiarrythmic effect and filed a patent application (Japanese Patent Laid-Open No. 158190/1985):

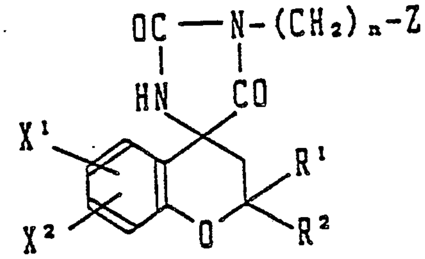

(A)

wherein $X^1$ and $X^2$ may be the same or different from each other and each represent a hydrogen atom, a halogen atom, a lower alkyl group, a lower alkoxy group or a nitro group or they form an alkylenedioxy group together with the adjacent carbon atom, $R^1$ and $R^2$ may be the same or different from each other and each represent a hydrogen atom or a lower alkyl group or $R^1$ and $R^2$ may form a cycloalkyl group having 5 or 6 carbon atoms together with the carbon atom to which they are bonded. Z represents a group of the fomula:

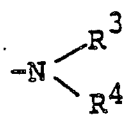

in which $R^3$ and $R^4$ may be the same or different from each other and each represent a hydrogen atom, a lower alkyl group, a hydroxyalkyl group or a substituted or unsubstituted aralkyl group or $R^3$ and $R^4$ may form a saturated heterocyclic group having 5 or 6 carbon atoms together with the nitrogen atom to which they are bonded, the heterocyclic group may further contain an oxygen atom,

a group of the formula:

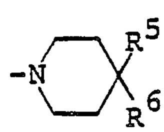

in which $R^5$ and $R^6$ may be the same or different from each other and each represent a hydrogen atom, a phenyl group or a group of the formula: $-OR^7$ ($R^7$ being a hydrogen atom, an acyl group, a lower alkyl group or a substituted or unsubstituted aralkyl group) or $R^5$ and $R^6$ may form a four-membered or five-membered cyclic group containing two oxygen atoms together with the carbon atom to which they are bonded or $R^5$ and $R^6$ may together form a group of the formula: $=O$, or

a group of the formula:

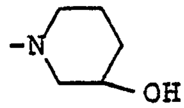 ,

and n represents an integer of 2 to 7.

The compounds of the above general formula have a characteristic feature that they have no heart depressant effect. Further the antiarrhythmic effect is long lasting and they have a low toxicity and a wide safety zone. Therefore, they are quite excellent antiarrythmic agents.

Thereafter the inventors made further investigations of 6-halogeno-2,2-dimethyl-1′-[3-(4-hydroxypiperidino)propyl]spiro[chroman-4,4′-imidazolidine]-2′, 5′-dione among the hydantoin derivatives of the above general formula (A).

The above-described compounds have an asymmetric carbon atom in the molecule and, therefore, they have d- and ℓ-optical isomers. The inventors investigated these isomers.

Namely, dℓ-6-halogeno-2,2-dimethylspiro[chroman-4,4′-imidazolidine]-2′,5′-dione produced by, for example, the process disclosed in the above-described Japanese Patent Laid-Open No. 158190/1985 was optically resolved to obtain the corresponding ℓ- and d-compounds, from which a corresponding ℓ-6-halogeno-2,2-dimethyl-1′-[3-(4-hydroxypiperidino)propyl]spiro[chroman-4,4′-imidazolidine]-2′,5′-dione and corresponding d-compound were obtained by the process disclosed in that Japanese Patent Laid-Open No. 158190/1985. Surprisingly, the ℓ- and d-compounds, particularly the ℓ-compound, were superior to the dl-compouond in view of, for example, the medicinal effect.

( Summary of the Invention )

After intensive investigations made over a long time under these circumstances, the inventors have found that optically active hydantoin derivatives of following formula (I) and acid addition salts thereof have an excellent antiarrythmic effect and have completed the present invention on the basis of this finding:

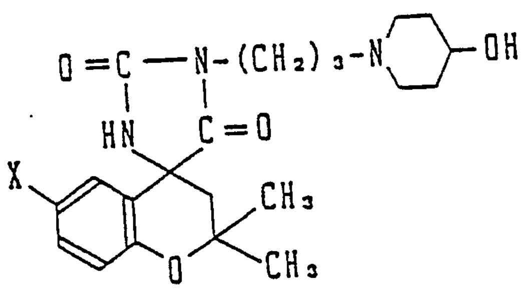

( I )

wherein X represents a halogen atom.

The term 'halogen atom' in the definition of X include chlorine, bromine and fluorine atoms. Among them, the most preferred halogen atom is chlorine.

The invention provides an optically active hydantoin compound, ℓ-6-halogen-2,2-dimethyl-1′-(3-(4-hydroxypiperidino)-propyl)spiro(chroman-4,4′-imidazolidine)-2′,5′-dione, having the formula [I] in which X is a halogen, and a pharmacologically acceptable acid addition salt thereof.

The invention provides a pharmaceutical composition which comprises a therapeutically effective amount of the compound or the salt as defined above and a pharmacologically acceptable carrier and then a method for treating arrhythmia by administering a therapeutically effective amount of the compound and the salt as defined above to a human being suffering from arrhythmia.

When X is a chlorine atom, the optically active compound is ℓ-6-chloro-2,2- dimethyl-1′-[3-(4-hydroxypiperidino)propyl]spirol[chroman-4,4′-imidazolidine]-2′,5′-dione or d-6-chloro-2,2-dimethyl-1′-[3-(4-hydroxypiperidino)propyl]sopiro[chroman-4,4′-imidazolidine]-2′,5′-dione. The ℓ-compound is superior to the d-compound as the antiarrythmic agent.

The acids of the pharmacologically acceptable acid addition salts of the optically active hydantoin derivatives of the present invention include, for example, inorganic acids such as phosphoric acid, hydrochloric acid, sulfuric acid, hydrogen bromide and hydrogen iodide as well as organic acids such as fumaric acid, oxalic acid, acetic acid, methanesulfonic acid, lactic acid, citric acid, tartaric acid and succinic acid.

The optically active hydantoin derivatives (I) of the present invention can be produced by, for example, optically resolving a dℓ-6-halogeno-2,2-dimethylspiro[chroman-4,4′-imidazolidine]-2′, 5′-dione produced by the process disclosed in the above-described Japanese Patent Laid-Open No. 158190/1985 and treating the obtained compound by the process disclosed therein. Various other processes can also be employed. As example of them is as follows:

d- or ℓ-compound of the formula:

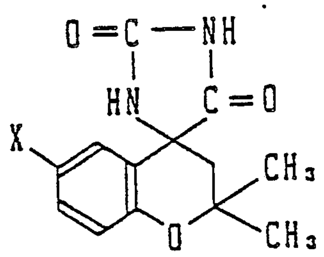

(II)

+

$Y-(CH_2)_3-Z$ (III)

wherein Y and Z each represent a halogen atom

+

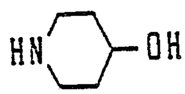

(IV)

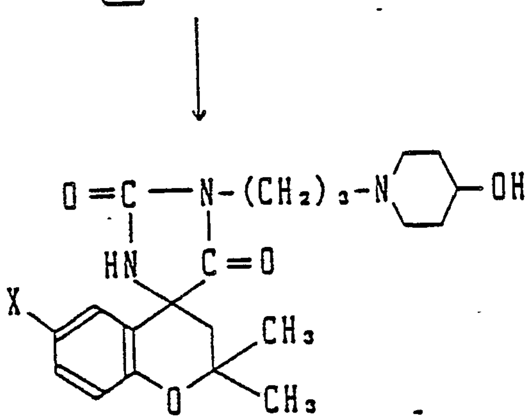

(I)

Namely in this process, the dℓ-6-halogeno-2,-2-dimethylspiro[chroman-4,4′-imidazolidine]-2′,5′-dione is optically resolved to obtain d- or ℓ-6-halogeno-2,2-dimethylspiro[chroman-4,4′-imidazolidine]-2′,5′-dione(II), which is then reacted with a dihalide compound of general formula (III) and the product thus formed is subjected to a condensation reaction with 4-hydroxypiperidine of general formula (IV) in the presence of a base in an ordinary manner to obtain the intended optically active hydantoin derivative.

The condensation reaction is conducted in the presence of a base such as potassium carbonate, sodium carbonate, triethylamine, sodium hydride or a sodium alkoxide in a solvent such as methanol, ethanol, isopropanol, butanol, dimethylacetamide or dimethylformamide at room temperature to 100 °C to form the intended product (I).

The dℓ-6-halogeno-2,2-dimethylspiro[chroman-4,4′-imidazolidine]-2′,5′-dione is optically resolved by, for example, the following process: the dℓ-compound is treated with a quaternary cinchonidine base or brucine in an ordianry manner to obtain N-methyl-cinchonidium salt or brucine salt of the compound. The salt is recrystallized in an ordinary manner and the obtained optically active salt is made free with an acid to obtain the d- or l-comound.

Givineg one example therefor, the dl-compound is dissolved in an organic solvent such as methanol or acetone. An equivalent amount of a solution of cinchonidine methohydroxide in methanol is added to the solution. The resulting solution is concentrated under reduced pressure to form amorphous N-methylcinchonidium salt of that compound. The amorphous compound is dissolved in acetone and the solution is left to stand to form crystals, namely N-methylcinchonidium salt of mainly d-6-chloro-2,2-dimethylsopiro-[chroman-4,4′-imidazolidine]-2′,5′-dione. The mother liquor is a solution rich in the salt of the ℓ-compound.

The crystals of the salt of mainly the d-compound is treated with hydrochloric acid and then recrystallized from an organic solvent in an ordinary manner to obtain d-6-chloro-2,2-dimethylspiro-[chroman-4,4′-imidazolidine]-2′,5′-dione.

On the other hand, the mother liquor rich in the salt of the ℓ-compound is treated with hydrochloric acid and then crystallized from an organic solvent in an ordinary manner to obtain 6-chloro-2,2-dimethylspiro-[chroman-4,4′-imidazolidine]-2′,5′-dione in which the amount of the ℓ-compound is a little larger than that of the d-compound. The mother liquor is recrystallized to obtain ℓ-6-chloro-2,2-dimethylspiro[chroman-4,4′-imidazolidine]-2′,5′-dione.

The optical resolution of the racemic mixture of the d-compound and the l-compound can be effected with use of brucine, for example. The mixture of the d-and l-compounds is dissolved in an organic solvent such as methanol and ethanol. An equivalent amount of brucine is added to the solution. The resulting mixture is concentrated at a reduced pressure and the concentrated product is mixed with diethylether to produce crystals. The crystals are then treated with hydrochloric acid and recrystallized with an organic solvent in the same manner as shown above with the quaternary cinchonidine to produce ℓ-6-chloro-2,2-dimethylspiro(chroman-4,4′-imidazolidine)-2′,5′-dione.

The antiarrythmic activity of the compound of the present invention was examined with aconitine-induced arrhythmia model of mice. When 0.1 mg/kg of aconitine is intraperitoneally injected, usually ventricular tachycardia occurs within 20 minutes. The compound of the present invention (d- or ℓ-compound) was orally administered one hour before the intraperitoneal injection of 0.1 mg/kg of aconitine and the tachycardia and the frequency of ventricular extrasystole were determined from the electrocardiogram to determine the antiarrhythmic activity ($ED_{50}$). The $ED_{50}$ of the d-compound was 40 mg/kg and that of the ℓ-compound was 5 mg/kg.

In the acute toxicity tests with mice, $LD_{50}$ of the d-compound was 700 mg/kg and that of the ℓ-compound was 200 mg/kg.

In the above-described experiments, it was confirmed that the compounds of the present invention, particularly the ℓ-compounds, have an excellent, long lasting antiarrhythmic activity, a low toxicity and a wide safety zone and, therefore, they are useful as a quite preferred antiarrythmic agent.

To elucidate the mechanism of the antiarrhythmic effect of the compounds of the present invention, the effects thereof on the resting potential and action potential of extirpated heart muscles of guinea pigs and pigs were examined by a microelectrode method. Although the compounds of the present invention exerted no influence on the resting potential, they reduced the rising rate of the action potential. Particularly under high electric stimulation conditions, the effect of inhibiting the action potential was strong. This property is common to antiarrythmic agents of Class I (such as quinidine and disopyramide). This suggests that the mechanism of the effect of the compounds of the present invention and the diseases (supraventricular and ventricular arrythmia) for which they are efficaceous are common to other antiarrhythmic agents of Class I.

One of the pharmacological features of the compounds of the present invention is that they inhibit myocardial contraction only very slightly. This is a quite desirable property when they are used as the antiarrhythmic agents. In particular, the compounds of the present invention exert no influence on the myocardial contraction even in an amount 10 times as much as the antiarrhythmically effective amount thereof, while quinidine and disopyramide reduce myocardial contraction even in an amount several times as much as the antiarrhythmically effective amount thereof.

The compounds of the present invention are usable as the antiarrhythmic agent effective for the treatment and prevention of various types of arrhythmia such as ventricular arrhythmia and atrial arrhythmia.

When the compound of the present invention is to be used as the antiarrhythmic agent, it is given by either oral or parenteral administration (such as intramuscular, subcutaneous or intravenous administration). The dose of the compound is not particularly limited, since it varies depending on the disease, symptoms, age, etc. Usually it is administered in an amount of about 1 to 1,000 mg/day, preferably about 100 to 300

mg/day in 1 to 3 portions to adults.

Preparations comprising the compound of the invention may be made in the form of tablets, granules, powders, capsules, injections, suppositories, etc. They are prepared by an ordinary process known in the art.

In producing an oral solid preparation, the active ingredient is combined with an excipient and, if necessary, a binder, disintegrator, lubricant, colorant, corrigent, etc. and then the mixture is shaped into tablets, coated tablets, granules, powder or capsules.

The excipients usable herein include, for example, lactose, corn starch, white sugar, glucose, sorbitol and crystalline cellulose. The binders include, for example, polyvinyl alcohol, polyvinyl ether, ethylcellulose, methylcellulose, acacia, tragacanth, gelatin, shellac, hydroxypropylcellulose, hydroxypropyl starch and polyvinylpyrrolidone. The disintegrators include, for example, starch, agar, gelatin powder, crystalline cellulose, calcium carbonate, sodium hydrogencarbonate, calcium citrate, dextrin and pectin. The lubricants include, for example, magnesium stearate, talc, polyethylene glycol, silica and hardened vegetable oils. The colorants include those permitted as colorants for medicines. The corrigents include, for example, cocoa powder, menthol, aromatic powder, peppermint oil, broneol and cinnamon powder. As a matter of course, the tablets and granules can be suitably coated with sugar, gelatin or the like.

The injection is prepared by adding, if necessary, a pH adjustor, buffering agent, stabilizer, solubilizer and preservative to the active ingredient and the mixture is treated in an ordinary manner to form a subcutaneous, intramuscular or intravenous injection.

[Example]

The following typical Example will further illustrate the present invention, which by no means limit the invention.

Example

(1) Production of cinchonidine methiodide

100 g of cinchonidine was dissolved in 1.6 ℓ of ethanol. 76 g of methyl iodide was added to the solution and the mixture was left to stand overnight. Crystals thus formed were recovered by filtration to obtain 127 g of cinchonidine methiodide.

(2) Production of solution of cinchonidine methohydroxide in methanol

109 g of cinchonidine methiodide was dissolved in 2 ℓ of methanol. The solution was passed through a column packed with 1 kg of Amberlyst A 27. The column was washed with 2.5 ℓ of methanol. The washing was combined with the eluate.

(3) ℓ-6-Chloro-2,2-dimethylspiro[chroman-4,4′-imidazolidine]-2′,5′-dione

70 g of dℓ-6-chloro-2,2-dimethylspiro[chroman-4,4′-imidazolidine]-2′,5′-dione was dissolved in 3.5 ℓ of methanol. The solution was added to the solution of cinchonidine methohydroxide in methanol prepared in above-described step (2). Methanol was distilled off under reduced pressure. The residue was crystallized from acetone. About 50 g of the crystals were filtered out. The filtrate was made acidic with an aqueous hydrochloric acid solution. After extraction with ethyl acetate followed by drying over sodium sulfate, a part of the solvent was distilled off under reduced pressure and about 24 g of crystals thus formed were filtered out. The filtrate was distilled under reduced pressure until the volume thereof was reduced to about 30 mℓ. It was left to stand under cooling with ice for 2 h and crystals thus formed were recovered by filtration (yield: 7.0 g). The filtrate was further distilled under reduced pressure and then cooled with ice to form crystals (yield: 1.5 g). 7.0 g and 1.5 g of the crystals thus obtained were combined and recrystallized from ethyl acetate.
Yield: 6.7 g

[α]D: -54.0° (C = 1.0 in MeOH)
Melting point: 236 to 237° C.

(4) ℓ-6-Chloro-2,2-dimethyl-1′-[3-(4-hydroxypiperidino)propyl]spiro[chroman-4,4′-imidazolidine]-2′,5′-dione

10 g of ℓ-6-chloro-2,2-dimethylspiro[chroman-4,4′-imidazolidine]-2′,5′-dione and 11 g of 1-bromo-3-chloropropane were dissolved in DMF. 1.6 g of sodium hydride (60 % suspension in a mineral oil) was added to the solution at room temperature. The reaction was conducted at a temperature elevated to 70° C for 15 min. 11 g of 4-hydroxypiperidine and 20 g of potassium carbonate were added to the reaction liquid and the mixture was heated at 100° C to conduct the reaction for 50 min. Water was added to the reaction mixture. After extraction with ethyl acetate, the extract was dried over sodium sulfate. The solvent was distilled off under reduced pressure and the residue was purified through a silica gel column to obtain 9.0 g of an oily product. After crystallization from ethyl acetate/hexane, the intended product was obtained.
Yield: 7.6 g
[α]D: -36.0° (C = 1 in MeOH)
Melting point: 164 to 165.5° C

(5) d-6-Chloro-2,2-dimethylspiro[chroman-4,4′-imidazolidine]-2′,5′-dione

140 g of dℓ-6-chloro-2,2-dimethylspiro[chroman-4,4′-imidazolidine]-2′,5′-dione was dissolved in methanol. The solution was added to a solution of 163 g of cinchonidine methohydroxide in methanol. Methanol was distilled off under reduced pressure and the product was crystallized from acetone (yield: 94 g). The crystals were dissolved in methanol, made acidic with hydrochloric acid and extracted with ethyl acetate. After drying over magnesium sulfate, the solvent was distilled off under reduced pressure and the residue was recrystallized from ethyl acetate.
Yield: 23.8 g
[α]D: +56.9° (C = 1.0 in MeOH)
Melting point: 238 to 239.5° C

(6) d-6-Chloro-2,2-dimethyl-1′-[3-(4-hydroxypiperidino)propyl]spiro[chroman-4,4′-imidazolidine]-2′,5′-dione

The same procedure as that of above-described step (4) was repeated except that 12 g of d-6-chloro-2,2-dimethylspiro[chroman-4,4′-imidazolidine]-2′,5′-dione was used.
Yield: 9.0 g
[α]D: +35.7° (C = 1.0 in MeOH) Melting point: 165.5 to 167° C

(7) ℓ-6-chloro-2,2-dimethylspiro(chroman-4,4′-imidazolidine)-2′,5′-dione is synthesized in another way.

282 g of dl-6-chloro-2,2-dimethylspiro(chroman-4,4′-imidazolidine)-2′,5′-dione and 431 mg of brucine dihydrate were dissolved in 10 ml of methanol. About 8 ml of the methanol was distilled out and the residue was mixed with about 8 ml of diethylether to produce crystals. 280 mg of the crystals were collected with filtration and dissloved in water. The solution was rendered acidic with a diluted hydrochloric acid and the intended product was extracted with ethyl acetate. The extracted liquid was dried with magnesium sulfate and almost all of the ethyl acetate was distilled out to obtain 90 mg of the crystals. The obtained crystals were found to have the same optical rotation and melting point as the product (3) above shown.

**Claims**

1. An optically active hydantoin compound, ℓ-6-halogen-2,2-dimethyl-1′-(3-(4-hydroxypiperidino)-propyl)spiro(chroman-4,4′-imidazolidine)-2′,5′-dione, having the formula:

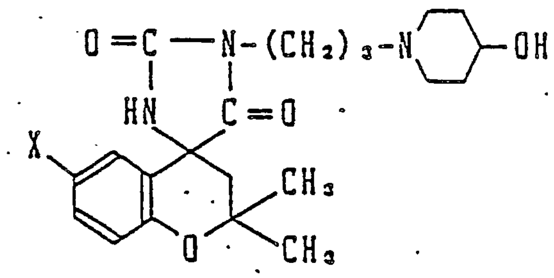

in which X is a halogen, and
a pharmacologically acceptable acid addition salt thereof.

2. The compound and the salt as claimed in Claim 1, in which X is chlorine.

3. The compound and the salt as claimed in Claim 1, in which the compound is
l-6-chloro-2,2-dimethyl-1′-[3-(4-hydroxypiperidino)propyl]spiro[chroman-4,4′-imidazolidine]-2′,5′-dione.

4. A process for the preparation of the compound as claimed in Claim 1 wherein the dl-6-halogeno-2,2-dimethyl-spiro[chroman-4,4′-imidazolidine]-2′,5′-dione is optically resolved to obtain d- or 1-6-halogeno-2,2-dimethylspiro[chroman-4,4′-imidazolidine]-2′,5′-dione (II)

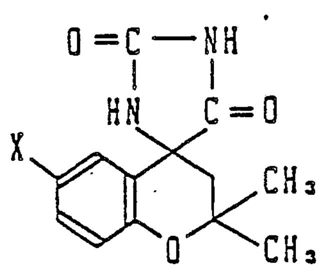

(II)

which is then reacted with a dihalide compound of general formula (III)
$Y-(CH_2)_3-Z$ (III)
wherein Y and Z each represent a halogen atom, and the product thus formed is subjected to a condensation reaction with 4-hydroxypiperidine of general formula (IV)

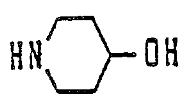

(IV)

in the presence of a base to obtain the optically active hydantoin derivative of the formula (I).

5. A pharmaceuticl composition which comprises a therapeutically effective amount of the compound or the salt as defined in Claim 1 and a pharmacologically acceptable carrier.

6. Use of a compound as claimed in any of the Claims 1 to 3 for making a medicament effective in the prevention and treatment of various types of arrhythmia.

European Patent Office

# EUROPEAN SEARCH REPORT

Application number

EP 89110780.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | DE - A1 - 3 503 074 (EISAI CO) * Claims 1,6,9; page 40, lines 1-20 * -- | 1-6 | C 07 D 491/107<br>A 61 K 31/415 |
| X | CHEMICAL ABSTRACTS, vol. 107, no. 15, October 12, 1987, Columbus, Ohio, USA EISAI CO. "Preparation of spiro(chroman-4,4'-imidazolidine)-2',5'-dione derivatives as antiarrhythmics" page 724, column 1, Abstract-no. 134 304p & Jpn. Kokai Tokkyo Koho JP 62 26 282 (87 26 282) -- | 1-6 | |
| X | CHEMICAL ABSTRACTS, vol. 106, no. 13, March 30, 1987, Columbus, Ohio, USA SAWADA, K. et al. "Voltage- and rate-dependent depression of V max of action potentials by a new antiarrhythmic agent, E-0747, in swine cardiac Purkinje fibers" page 46, column 1, Abstract-no. 95 845y & J. Cardiovasc. Pharmacol. 1987, 9(1), 51-6 -- | 1-6 | TECHNICAL FIELDS SEARCHED (Int. Cl.4)<br>C 07 D 491/00 |
| X | CHEMICAL ABSTRACTS, vol. 107, no. 11, September 14, 1987, Columbus, Ohio, USA MITSUHASHI, H. et al. "Antiarrhythmic effects of a new drug, E-0747, on canine ventricular arrhythmia models" page 46, column 1, Abstract- | 1-6 | |

The present search report has been drawn up for all claims

| Place of search | Date of completion of the search | Examiner |
|---|---|---|
| VIENNA | 02-08-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82

European Patent Office

# EUROPEAN SEARCH REPORT

Application number

-2-

EP 89110780.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | -no. 89 540z & Jpn. J. Pharmacol. 1987, 44(2), 155-62 ---- | | |
| | | | TECHNICAL FIELDS SEARCHED (Int. Cl.4) |

The present search report has been drawn up for all claims

| Place of search | Date of completion of the search | Examiner |
|---|---|---|
| VIENNA | 02-08-1989 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82